# EUROPEAN PATENT SPECIFICATION

(11) **EP 2 688 578 B1**
(45) Date of publication and mention of the grant of the patent: **27.03.2019**
(21) Application number: 12710120.2
(22) Date of filing: 12.03.2012
(51) Int. Cl.: A61K 38/08, A61K 38/10, A61K 39/395, A61P 17/00, C07K 16/28, C07K 7/06, C07K 7/08, C07K 16/30, G01N 33/68

(54) **MIA (MELANOMA INHIBITORY ACTIVITY) INHIBITORS FOR PREVENTING AND CURING VITILIGO**
INHIBITOREN DES MIA-(MELANOMA INHIBITORY ACTIVITY-)PROTEINS ZUR VORBEUGUNG UND BEHANDLUNG VON VITILIGO
INHIBITEURS DE MIA (ACTIVITÉ INHIBITRICE DU MÉLANOME) DESTINÉS À PRÉVENIR ET TRAITER LE VITILIGO

(30) Priority: 22.03.2011 IT RM20110134
(43) Date of publication of application: 29.01.2014
(73) Proprietor: Bordignon, Matteo, 35126 Padova (IT)
(72) Inventor: Bordignon, Matteo, 35126 Padova (IT)
(74) Representative: Currado, Luisa
(86) International application number: PCT/IB2012/051141
(87) International publication number: WO 2012/127352

(56) References cited:
- WO-A1-03/064457
- US-A- 5 973 123
- KUMAR R ET AL: "Role of apoptosis and melanocytorrhagy: a comparative study of melanocyte adhesion in stable and unstable vitiligo", BRITISH JOURNAL OF DERMATOLOGY, vol. 164, no. 1, January 2011 (2011-01), pages 187-191, XP002660948,
- SASAHIRA TOMONORI ET AL: "High mobility group box-1-inducible melanoma inhibitory activity is associated with nodal metastasis and lymphangiogenesis in oral squamous cell carcinoma.", CANCER SCIENCE SEP 2008 LNKD- PUBMED:18616526, vol. 99, no. 9, September 2008 (2008-09), pages 1806-1812, XP002660949, ISSN: 1349-7006
- BORDIGNON MATTEO ET AL: "Expression of MIA (melanoma inhibitory activity) in vitiliginous melanocytes: a novel pathogenetic pathway?", JOURNAL OF INVESTIGATIVE DERMATOLOGY, vol. 131, no. Suppl. 2, September 2011 (2011-09), page S112, XP002660950, & 41ST ANNUAL MEETING OF THE EUROPEAN-SOCIETY-FOR-DERMATOLOGICAL-RESEA RCH; BARCELONA, SPAIN; SEPTEMBER 07 -10, 2011
- ALIKHAN A ET AL: "Vitiligo: A comprehensive overview: Part I. Introduction, epidemiology, quality of life, diagnosis, differential diagnosis, associations, histopathology, etiology, and work-up", JOURNAL OF THE AMERICAN ACADEMY OF DERMATOLOGY 2011 MOSBY INC. USA LNKD- DOI:10.1016/J.JAAD.2010.11.061, vol. 65, no. 3, September 2011 (2011-09), pages 473-491, XP002660951, ISSN: 0190-9622

## Description

The present invention refers to the field of pharmaceuticals, in particular preparations for detecting,curing non-segmental vitiligo.

### Background of the invention

Vitiligo, also named as common generalized vitiligo, is an acquired pigmentary disorder of the skin and mucous membranes, and it is characterized by circumscribed depigmented macules and patches. Vitiligo is a progressive disorder in which some or all of the melanocytes in the affected skin seem to be selectively destroyed. Vitiligo affects 0.5-2% of the world population, and the average age of onset is 20 years.

Non-segmental vitiligo is the most common sub-type of vitiligo. Non-segmental vitiligo is an acquired chronic pigmentation disorder characterized by white patches, often symmetrical, which usually increase in size with time, corresponding to a substantial loss of functioning epidermal and sometimes hair follicle melanocytes *(*Taieb A, Picardo M; VETF Members. The definition and assessment of vitiligo: a consensus report of the Vitiligo European Task Force. Pigment Cell Res 2007; 20: 27-35*).*

Despite many studies performed on vitiligo skin, the exact pathogenesis of this dermatosis is still to be clarified and several pathogenetical mechanisms have been proposed during years, involving autoimmunity, cytotoxic metabolites, neural and genetic components; no convincing model describing the interplay of all these contributing factors has been formulated *(*Schallreuter KU, Bahadoran P, Picardo M, et al. Vitiligo pathogenesis: autoimmune disease, genetic defect, excessive reactive oxygen species, calcium imbalance, or what else? Exp Dermatol 2008; 17: 139-40*).* So far, two main features can be unequivocally ascribed to vitiligo: histologically there is lack of inflammation and clinically there is association with other autoimmune disorders. Recently, it has been suggested that the major and possible primary predisposing factor in vitiligo development could be a defective adhesion of melanocytes and that, based on *in vivo* and *in vitro* observations *(*Gauthier Y, Cario-Andre M, Lepreux S, Pain C, Taieb A. Melanocyte detachment after skin friction in non lesional skin of patients with generalized vitiligo. Br J Dermatol 2003; 148: 95-101*;* Cario-Andre' M, Pain C, Gauthier Y, Taïeb A. The melanocytorrhagic hypothesis of vitiligo tested on pigmented, stressed, reconstructed epidermis. Pigment Cell Res 2007; 20: 385-393*),* mechanical trauma and various chemical stressors could represent the main precipitating events. This theory considers vitiligo as a disease caused by the chronic detachment and named melanocytorrhagy the trans-epidermal loss of melanocytes *(*Gauthier Y, Cario-Andre M, Lepreux S, Pain C, Taieb A. Melanocyte detachment after skin friction in non lesional skin of patients with generalized vitiligo. Br J Dermatol 2003; 148: 95-101*;* Cario-Andre' M, Pain C, Gauthier Y, Taïeb A. The melanocytorrhagic hypothesis of vitiligo tested on pigmented, stressed, reconstructed epidermis. Pigment Cell Res 2007; 20: 385-393*;* Gauthier Y, Cario Andre M, Taïeb A. A critical appraisal of vitiligo etiologic theories. Is melanocyte loss a melanocytorrhagy? Pigment Cell Res 2003; 16: 322-32*),* which would be the silent manner by which the vitiliginous skin eliminates its pigment away.

Several therapies can be considered in treatment of vitiligo *(*Sehgal VN, Srivastava G. Vitiligo treatment options: an evolving scenario. J Dermatolog Treat 2006; 17: 262-75*).* None of the single vitiligo therapies produces predictably good results in all patients; the response to single therapy is highly variable. Generally, the treatment must be individualized, and patients should be made aware of the risks associated with therapy. The most common treatments for non-segmental vilitigo are:
- Narrow-Band Ultra Violet B (UVB-NB) phototherapy: widely used with good clinical results, based on narrow-band fluorescent tubes with an emission spectrum of 310-315 nm and a maximum wavelength of 311 nm. Treatment frequency is 2-3 times weekly, but never on consecutive days. This treatment can be safely used in children, pregnant women, and lactating women. Short-term adverse effects include pruritus and xerosis. Long-term adverse effects are not well defined since the carcinogenic potential of UVB is still to be clarified. The therapy achieved the best results on vitiligoid patches of face and trunk; very poor results usually are achieved on vitiligo of hands and feet.
- Corticosteroid therapy: corticosteroids are used topically, systemically o intralesionally; topical steroids are often chosen first to treat localized vitiligo but the results of therapy have been reported as moderately successful, particularly in patients with localized vitiligo and/or an inflammatory component to their vitiligo, even if the inflammation is subclinical. The use of topic, systemic or intra-lesional steroids may have side effects like toxicity (systemic) or cutaneous atrophy (topical or intra-lesional); in addiction very poor results usually are achieved on vitiligo of hands and feet.
- Topical calcineurin inhibitors (tacrolimus and pimecrolimus): tacrolimus and pimecrolimus could be successful used to cure vitiligoid patches of face and neck; recently, Food and Drug Administration (FDA) imposed a "black box warning" on the topical calcineurin inhibitors, because of a theoretic risk of oncogenesis because of the trivial systemic absorption of these agents; in addiction very poor results usually are achieved on vitiligo of hands and feet.

Surgical alternatives exist for the treatment of vitiligo; however, because of the time-consuming nature of surgical therapies, these treatment regimens are limited to segmental or localized vitiligo.

So far, anyway, none of these therapies could reach a complete re-pigmentation of the vitiliginous patches in all the patients.

The precise mechanism of action of these therapies is still unknown and it is generically attributed to their immunosuppressive activity. It is remarkable, however, that UVB-NB radiation increase alpha5beta1 integrin expression on melanocytes after exposure *(*Neitmann M, Alexander M, Brinckmann J, Schlenke P, Tronnier M. Attachment and chemotaxis of melanocytes after ultraviolet irradiation in vitro. Br J Dermatol 1999; 141: 794-801*).*

A particular subgroup of integrins, named alpha5beta1, seems to be involved in melanocyte adhesion and in vitiligo therapy *(*Swope VB, Supp AP, Schwemberger S, Babcock G, Boyce S. Increased expression of integrins and decreased apoptosis correlate with increased melanocyte retention in cultured skin substitutes. Pigment Cell Res 2006 ; 19: 424-33*).* Interactions between melanocytes and the basement membrane are mediated by integrins *(*Hara M, Yaar M, Tang A, Eller MS, Reenstra W, Gilchrest BA. Role of integrins in melanocyte attachment and dendricity. J Cell Sci 1994; 107: 2739-48*)* and many therapies useful in this dermatosis seem to be effective in the expression of these molecules *(*Neitmann M, Alexander M, Brinckmann J, Schlenke P, Tronnier M. Attachment and chemotaxis of melanocytes after ultraviolet irradiation in vitro. Br J Dermatol 1999; 141: 794-801*).*

Active detachment and alpha5beta1 integrins are very interesting features also in another and more severe melanocytic disorder such as malignant melanoma. Malignant melanoma is the most severe skin cancer and its metastatic form is associated with the poorest prognosis *(*Thompson JF, Scolyer RA, Kefford RF. Cutaneous melanoma. Lancet 2005; 365 :687-701*).*

Recently, it seems that the metastatic dissemination of malignant melanoma (a melanocyte-derived tumor) could be mediated by an active detachment of tumor cells and that this detachment can be caused by a small protein secreted from malignant melanoma cells called melanoma inhibitory activity - MIA *(*Bosserhoff AK, Stoll R, Sleeman JP, Bataille F, Buettner R, Holak TA. Active detachment involves inhibition of cell-matrix contacts of malignant melanoma cells by secretion of melanoma inhibitory activity. Lab Invest 2003; 83: 1583-94*).* It has been demonstrated that MIA interacts with integrin alpha5beta1 *(*Bauer R, Humphries M, Fassler R, Winklmeier A, Craig SE, Bosserhoff AK. Regulation of integrin activity by MIA. J Biol Chem 2006; 281: 11669-77*).* So it seems that the interactions between a malignant melanocytes self-produced protein (MIA) and a self-melanocyte adhesion molecule (alpha5beta1 integrin) would be responsible of an active detachment of neoplastic melanocyte cells in malignant melanoma.

WO 03/64457 discloses peptides, modified peptides and antibody or antibody fragment inhibiting the activity of MIA and their use for treating solid tumors, leukemia and degenerative disorders.

The technical problem of the present invention is to provide compounds which avoid the side effects of the known therapies, such as pruritus and xerosis or cutaneous atrophy, toxicity and potential carcinogenesis or oncogenesis.

At the same time is highly desirable to provide compounds leading to complete re-pigmentation irrespective of the area to be treated, thus providing good results even on hands and feet, generally poorly recovered.

There is also a strong felt need for active agents capable to inhibiting the onset of the disease in patients being prone to it.

The same inventor surprisingly and unexpectedly found that MIA protein is present in patients affected by vitiligo and not in normal people and it is involved in the real pathogenetic mechanism which lead to formation of vitiliginous patches.

The presence of MIA explains why a clear clinical inflammation is lacking in vitiligo patches. The detachment of melanocyte does not activate the inflammation pathway, as there is no cellular necrosis or apoptosis near the basal membrane.

Nowadays, targeted therapies are not available for vitiligo as a target molecule has never been found.

MIA inhibitors represent a targeted therapy for non-segmental vitiligo.

Due to their specific binding to MIA, these molecules do not raise the side-effects commonly reported for actual therapies as cutaneous atrophy, pruritus, toxicity or oncogenetic potential.

Moreover, the inhibition of MIA, by avoiding damages towards melanocytes leads to re-pigmentation defiant of treated the anatomic area, thus implementing the treatment of difficult areas such as hands and feet.

The therapy-resistant site for vitiligo, such as hands and feet, are said to be due to the lack of melanocytes reservoir in these sites; our data shows a high presence of MIA against the melanocytes of these sites, probably due the continuous friction of these zones. Inhibitors of MIA, by removing all molecules of MIA, successfully treat these areas. Therefore peptides, modified peptides and inhibiting the activity of MIA can be used for treating non-segmental vitiligo by inducing re-pigmentation.

Said compounds can also be used useful also for preventing the appearance of vitiliginous patches.

Said compounds can also be used for making the already known therapies more effective. Is therefore object of the present invention the use of at least one peptide with sequence ID No. 1 to 49 for preventing and curing non-segmental vitiligo.

In a further object of the present invention, in said peptides one or more amino acid is substituted by a natural amino acid or a non-natural amino acid.

In a further object of the present invention, the non-natural amino acid is a modified natural amino acid wherein the modification is a substitution of one or more atoms with a functional group comprising 1 to 12 atoms selected from C, H, N, S, O, P, F, Cl, Br, I, Se. In a further object of the present invention, the above peptides comprise an additional amino acid ore one amino acid is deleted.

In a further object of the present invention, the modification of amino acid of the peptide with sequence ID No. 1 to 49 means glycosylation, acetylation, hydroxylation (hydroxyproline), carboxylation (gamma-carboxyglutamate), phosphorylation, alkylation, myristoylation (N-terminal), palmitoylation and prenylation) as well as non-naturally occurring amino acids including, trans-3- methylproline, 2, 4-methanoproline, cis-4-hydroxyproline, trans-4- hydroxyproline, N-methylglycine, allo-threonine, methylthreonine, hydroxyethylcysteine, hydroxyethylhomocysteine, nitroglutamine, homo- glutamin, pipecolic acid, tert-leucine, norvaline, 2-azaphenylalanine, 3- azaphenylalanine, 4 azaphenylalanine, and 4-fluorophenylalanine.

Further characteristics of the present invention would be clear from the following detailed description with reference to the experimental examples and the attached sheets of drawings.

### Brief Description of the figures

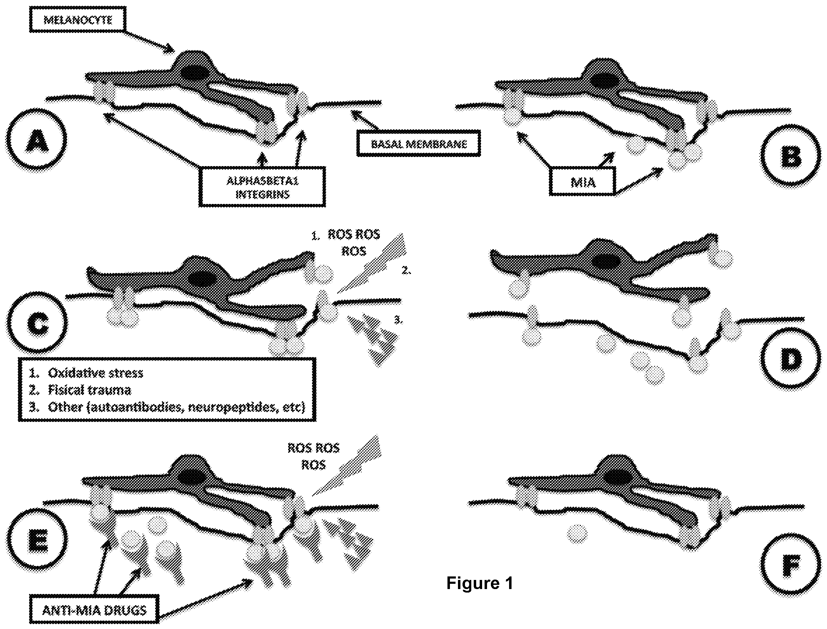

Figure 1 shows the mechanism of action of alpha5beta1 integrin and MIA in melanocytes leading to vitiligo. In panel A the normal adhesion of melanocyte with the basal membrane, mediated by alpha5beta1 integrins is shown. In panel B a vitiligious melanocyte attacked by MIA, which binds to alpha5beta1 integrins, is shown. In panel C: it is shown that, after the binding of MIA with alpha5beta1 integrins, the presence of other precipitating factors as oxidative stress, physical trauma or autoantibodies lead to a partial detachment of the melanocyte. In panel D the complete detachment of melanocyte and formation of vitiligoid patches on the skin (melanocytorrhagy) is shown. In panel E an anti-MIA drug binding to MIA and inactivating it is shown. In panel F the vitiliginous melanocyte keeping the adhesion with the basal membrane even in the presence of precipitating factors is shown.

### Detailed description of the Invention

### Definitions

Within the meaning of the present invention, vitiligo means an acquired progressive pigmentary disorder of the skin and mucous membranes characterized by circumscribed depigmented macules and patches.

Within the meaning of the present invention, a peptide inhibits the activity of MIA by binding to MIA.

Within the meaning of the present invention, natural amino acids are the 20 amino acids occurring in natural proteins and peptides.

Within the meaning of the present invention, non-natural amino acids are non-genetically-coded amino acids that either occur naturally or are chemically synthesized.

Within the meaning of the present invention, modified amino acids means amino acids modified by glycosylation, acetylation, hydroxylation (hydroxyproline), carboxylation (gamma-carboxyglutamate), phosphorylation,alkylation, myristoylation (N-terminal), palmitoylation and prenylation.

Object of the present invention is the use of peptides, modified peptides and antibody or antibody fragment, which inhibit the activity of MIA, for preventing and curing non-segmental vitiligo.

Said peptides inhibiting the activity of MIA are:
SEQ ID NO: 01 VPHIPPN
SEQ ID NO: 02 MPPTQVS
SEQ ID NO: 03 QMHPWPP
SEQ ID NO: 04 QPPFWQF
SEQ ID NO: 05 TPPQGLA
SEQ ID NO: 06 IPPYNTL
SEQ ID NO: 07 AVRPAPL
SEQ ID NO: 08 GAKPHPQ
SEQ ID NO: 09 QQLSPLP
SEQ ID NO: 10 GPPPSPV
SEQ ID NO: 11 LPLTPLP
SEQ ID NO: 12 QLNVNHQARADQ
SEQ ID NO: 13 TSASTRPELHYP
SEQ ID NO: 14 TFLPHQMHPWPP
SEQ ID NO: 15 VPHIPPNSMALT
SEQ ID NO: 16 RLTLLVLIMPAP
   (WO03064457; Stoll et al.,30 2001, EMBO J. 20: 340-349).
SEQ ID NO: 17 YNLPKVSSNLSP
SEQ ID NO: 18 MPPTQVSKFRLI
SEQ ID NO: 19 ANIDATPLFLRA
SEQ ID NO: 20 LLRTTETLPM FL
SEQ ID NO: 21 SALEPLV
SEQ ID NO: 22 GSPTPNA
SEQ ID NO: 23 APSHATH
SEQ ID NO: 24 TTVGHSD
SEQ ID NO: 25 THFSTFT
SEQ ID NO: 26 SLLLDTS
SEQ ID NO: 27 SVAMKAHKPLLP
SEQ ID NO: 28 NTIPGFASKSLD
SEQ ID NO: 29 VSNYKFYSTTSS
SEQ ID NO: 30 VSRHQSWHPHDL
SEQ ID NO: 31 HLNILSTLWKYR
SEQ ID NO: 32 HNASPSWGSPVM
SEQ ID NO: 33 SHPWNAQRELSV
SEQ ID NO: 34 HHWPFWRTLPLS
SEQ ID NO: 35 WHTKFLPRYLPS
SEQ ID NO: 36 NNTSFTVVPSVP
SEQ ID NO: 37 SHLSTWKWWQNR
SEQ ID NO: 38 FHWHPRLWPLPS
SEQ ID NO: 39 WHWTYGWRPPAM
SEQ ID NO: 40 FHWRYPLPLPGQ
SEQ ID NO: 41 WHWPLFIPNTTA
SEQ ID NO: 42 WHNGIWWHYGVR
SEQ ID NO: 43 HHLNYLWPWTRV
SEQ ID NO: 44 FWHRWSTFPEQP
SEQ ID NO: 45 WHMSYFWTRPPQ
SEQ ID NO: 46 FHLNWPSRADYL
SEQ ID NO: 47 WHKNTNWPWRTL
SEQ ID NO: 48ALSPSQSHPVRS
SEQ ID NO: 49 GTQSTAIPAPTD
   (WO03064457)

In the peptides from ID 1 to 49 one or more amino acid can be substituted by a natural or a non-natural amino acid.

The peptides from ID 1 to 49 can comprise an additional amino acid ore one amino acid is deleted.
the peptides from ID 1 to 49 may comprise at least one amino acid modified by glycosylation, acetylation, hydroxylation (hydroxyproline), carboxylation (gamma-carboxyglutamate), phosphorylation, alkylation, myristoylation (N-terminal), palmitoylation and prenylation) as well as non-naturally occurring amino acids including, trans-3-methylproline, 2, 4-methanoproline, cis-4-hydroxyproline, trans-4- hydroxyproline, N-methylglycine, allo-threonine, methylthreonine, hydroxyethylcysteine, hydroxyethylhomocysteine, nitroglutamine, homo- glutamin, pipecolic acid, tert-leucine, norvaline, 2-azaphenylalanine, 3- azaphenylalanine, 4 azaphenylalanine, and 4-fluorophenylalanine.

Are further objects of the present invention the pharmaceutical composition comprising at least one of the above peptides and/or at least one of the above anti-MIA antibodies solubilized and/or vehicle by pharmaceutically acceptable excipients and/or diluents.

### Example

Bioptic samples 10 bioptic samples were collected from edges injured areas of patients with conclamed non-segmental vitiligo, also samples of control skin were collected.

Macroscopically normal pigmented skins on the volar surface of the forearm of five healthy subjects were chosen for the experiments as sample controls.

All the patients have a negative history of melanoma and were screened with skin exams for absence of this cutaneous neoplasia.

### Histopathology and Immunohistochemistry

Macroscopic and microscopic analyses were performed on the pathologic and normal specimens. All samples were fixed in 10% formalin in 0.1-mol/L (pH = 7.4) phosphate buffer, dehydrated in a series of rising alcohol concentrations, and then embedded in paraffin for light microscopy. Four micrometer- thick sections were stained with hematoxylin and eosin, Heidenheim modified Azan-Mallory and examined by two independent pathologists.

Immunohistochemical staining with antibodies against leucocytes (CD45, clone 2B11+PD7/6.,dil 1/200, DAKO), lymphocytes (CD43 clone DF-T1 dil.1/200:DAKO, CD45RO clone CLB-UCHL1dil: Monosan, CD20 clone L26 dil:1/200 DAKO), and macrophages (CD68 clone KP1 dil 1/200 DAKO), were performed.

Sections obtained from each skin-biopsy were prepared, treated, and stained for immunohistochemistry according to standard procedures.

For the detection of premelanosomes and mature melanosomes we used S100 polyclonal rabbit antibody and HMB45 (clone HMB45 dil 1/100 DAKO).

Briefly, slides were treated with 0.3% hydrogen peroxide in methanol to block endogenous peroxidase activity, then washed with phosphate-buffered saline and incubated in buffered normal horse serum to prevent nonspecific Ab binding. Sections were incubated with the primary Abs for 1 hour at room temperature. After washing, a biotin-labeled secondary Ab was applied, followed by an avidin-peroxidase conjugate. Diaminobenzidine was used as a chromogen.

Two antibodies in two different sequential sections of patients and controls with anti-human integrin alpha5beta1 (Chemicon, by Millipore, MA, USA Clone JB 1/50), and melanoma inhibitory activity (MIA, Santa Cruz Biotechnology, CA, USA, polyclonal antibody IgG dil:1/20) were tested and visualized with peroxidase diaminobenzidine (DAB). Double immunostaining to illustrate co-localization of MIA protein with integrin alpha5beta1slides was done simultaneously on the same section with a sequential immuno-staining technique with anti-human integrin alpha5beta1 and melanoma inhibitory activity and visualizing the outcome with anti-mouse or anti-rabbit fluorescein isothiocyanate- or rhodamine-conjugated secondary antibodies (Chemicon, by Millipore, MA, USA). Nuclei were stained with TO-PRO 3 (Invitrogen, Molecular Probes; distributed by Invitrogen, Milan, Italy).

Microphotographs were taken using a TCS-SL laser scanner confocal microscope (Leica, Wetzar, Germany).

### Results

Nine on ten of the biotpic samples were positive for MIA (table 1). The only MIA-negative sample derived from a patient suffering from segmental vitiligo, whereas all the other MIA-positive samples were derived from patients suffering from non-segmental vitiligo. All skin samples used as control were negative for the presence of MIA.

The anti-alpha5beta1 antibody perfectly co-localized with anti-MIA antibody and that this feature where present also in melanocyte already detached from basal membrane and found in the upper epidermis.

**Table I**

| Patient number | Sex | Age | Type of vitiligo | Biopsy from | Presence of MIA |
|---|---|---|---|---|---|
| 1 | M | 45 | NON-SEGMENTAL | HAND | YES |
| 2 | F | 56 | NON-SEGMENTAL | ABDOMEN | YES |
| 3 | M | 62 | SEGMENTAL | BACK | NO |
| 4 | F | 35 | NON SEGMENTAL | LEG | YES |
| 5 | F | 59 | NON SEGMENTAL | ABDOMEN | YES |
| 6 | F | 40 | NON SEGMENTAL | BACK | YES |
| 7 | M | 48 | NON SEGMENTAL | ABDOMEN | YES |
| 8 | F | 58 | NON SEGMENTAL | LEG | YES |
| 9 | F | 32 | NON SEGMENTAL | BACK | YES |
| 10 | F | 61 | NON SEGMENTAL | BACK | YES |

The presence of MIA in non-malignant melanocytes was completely unexpected, as normal melanocytes have already been investigated for MIA with negative results.

The data demonstrate that MIA is involved in the pathogenetic mechanism of non-segmental vitiligo. The formation of the vitiliginous patches is consequent to a melanocytorrhagy attributed to the interaction of MIA with alpha5beta1 integrins, causing the elimination of pigments in vitiliginous skin.

The use of MIA inhibitors as targeted therapy for blocking the action of MIA lead to a complete re-pigmentation in every anatomic area.

MIA inhibitors prevent the development of vitiligo in patients already suffering of this dermatosis and in clinical remission.

The presence of MIA in vitiligoid melanocytes is an effective marker for histological definition of the disease in skin specimen.

## Claims

1. A MIA-inhibitor peptide selected from the group consisting of from SEQ ID NO: 1 to SEQ ID NO: 49 for use in preventing and curing non-segmental vitiligo.

2. Peptides for use according to claim 1, wherein in the peptides from SEQ ID NO: 1 to SEQ ID NO: 49 one or more amino acid can be substituted by a natural or a non-natural amino acid.

3. Peptides for use according to claim 1, wherein the peptides from SEQ ID NO: 1 to SEQ ID NO: 49 can comprise an additional amino acid.

4. Peptides for use according to claim 1, wherein in the peptides from SEQ ID NO: 1 to SEQ ID NO: 49 one amino acid is deleted.

5. Peptides for use according to claim 1, wherein the peptides from SEQ ID NO: 1 to SEQ ID NO: 49 comprise at least one amino acid modified by glycosylation, acetylation, hydroxylation (hydroxyproline), carboxylation (gamma-carboxyglutamate), phosphorylation, alkylation, myristoylation (N-terminal), palmitoylation and prenylation)

6. Peptides for use according to claim 1, wherein the peptides from SEQ ID NO: 1 to SEQ ID NO: 49 comprise at least one amino acid modified by non-naturally occurring amino acids selected from the group consisting of trans-3-methylproline, 2, 4-methanoproline, cis-4-hydroxyproline, trans-4-hydroxyproline, N-methylglycine, allo-threonine, methylthreonine, hydroxyethylcysteine, hydroxyethylhomocysteine, nitroglutamine, homo-glutamin, pipecolic acid, tert-leucine, norvaline, 2-azaphenylalanine, 3- azaphenylalanine, 4 azaphenylalanine, and 4-fluorophenylalanine.

7. Peptides for use according to anyone of claims 1-6, wherein they are provided as pharmaceutical composition with a pharmaceutically acceptable excipient.

## Patentansprüche

1. MIA-Hemmer-Peptid, ausgewählt aus der Gruppe, bestehend aus SEQ ID Nr. 1 bis SEQ ID Nr. 49 zur Verwendung bei der Verhinderung und Behandlung von nicht-segmentaler Vitiligo.

2. Peptide zur Verwendung gemäß Anspruch 1, worin in den Peptiden von SEQ ID Nr. 1 bis SEQ ID Nr. 49 eine oder mehrere Aminosäuren durch eine natürliche oder nicht-natürliche Aminosäure ersetzt sein können.

3. Peptide zur Verwendung gemäß Anspruch 1, worin die Peptide von SEQ ID Nr. 1 bis SEQ ID Nr. 49 eine zusätzliche Aminosäure enthalten können.

4. Peptide zur Verwendung gemäß Anspruch 1, worin in den Peptiden von SEQ ID Nr. 1 bis SEQ ID Nr. 49 eine Aminosäure deletiert ist.

5. Peptide zur Verwendung gemäß Anspruch 1, worin die Peptide von SEQ ID Nr. 1 bis SEQ ID Nr. 49 wenigstens eine Aminosäure enthalten, die modifiziert ist durch Glykosylierung, Acetylierung, Hydroxylierung (Hydroxyprolin), Carboxylierung (Gammacarboxyglutamat), Phosphorylierung, Alkylierung, Myristylierung (N-terminal), Palmitoylierung und Prenylierung.

6. Peptide zur Verwendung gemäß Anspruch 1, worin die Peptide von SEQ ID Nr. 1 bis SEQ ID Nr. 49 wenigstens eine Aminosäure enthalten, die durch nicht natürlich vorkommende Aminosäuren modifiziert ist, ausgewählt aus der Gruppe, bestehend aus trans-3-Methylprolin, 2,4-Methanoprolin, cis-4-Hydroxyprolin, trans-4-Hydroxyprolin, N-Methylglycin, allo-Threonin, Methylthreonin, Hydroxyethylcystein, Hydroxyethylhomocystein, Nitroglutamin, Homoglutamin, Pipecolinsäure, tert-Leucin, Norvalin, 2-Azaphenylalanin, 3-Azaphenylalanin, 4-Azaphenylalanin und 4-Fluorphenylalanin.

7. Peptide zur Verwendung gemäß einem der Ansprüche 1 - 6, worin diese als pharmazeutische Zusammensetzung mit einem pharmazeutisch annehmbaren Hilfsstoff bereitgestellt werden.

## Revendications

1. Peptide inhibiteur de MIA sélectionné dans le groupe constitué de SEQ ID NO : 1 à SEQ ID NO : 49 pour son utilisation dans la prévention et le traitement du vitiligo non segmenté.

2. Peptides pour leur utilisation selon la revendication 1, dans lesquels dans les peptides de SEQ ID NO : 1 à SEQ ID NO : 49 un acide aminé ou plus peut être substitué par un acide aminé naturel ou non naturel.

3. Peptides pour leur utilisation selon la revendication 1, dans lesquels les peptides de SEQ ID NO : 1 à SEQ ID NO : 49 peuvent comprendre un acide aminé supplémentaire.

4. Peptides pour leur utilisation selon la revendication 1, dans lesquels dans les peptides de SEQ ID NO : 1 à SEQ ID NO : 49 un acide aminé est délété.

5. Peptides pour leur utilisation selon la revendication 1, dans lesquels les peptides de SEQ ID NO : 1 à SEQ ID NO : 49 comprennent au moins un acide aminé modifié par glycosylation, acétylation, hydroxylation (hydroxyproline), carboxylation (gamma-carboxyglutamate), phosphorylation, alkylation, myristoylation (N-terminale), palmitoylation et prénylation.

6. Peptides pour leur utilisation selon la revendication 1, dans lesquels les peptides de SEQ ID NO : 1 à SEQ ID NO : 49 comprennent au moins un acide aminé modifié par des acides aminés non naturels sélectionnés dans le groupe constitué de la trans-3-méthylproline, de la 2,4-méthanoproline, de la cis-4-hydroxyproline, de la trans-4-hydroxyproline, de la N-méthylglycine, de l'allo-thréonine, de la méthylthréonine, de l'hydroxyéthylcystéine, de l'hydroxyéthylhomocystéine, de la nitroglutamine, de l'homo-glutamine, de l'acide pipécolique, de la tert-leucine, de la norvaline, de la 2-azaphénylalanine, de la 3-azaphénylalanine, de la 4-azaphénylalanine, et de la 4-fluorophénylalanine.

7. Peptides pour leur utilisation selon l'une quelconque des revendications 1 à 6, dans lesquels ils sont fournis sous la forme d'une composition pharmaceutique avec un excipient pharmaceutiquement acceptable.


### SEQUENCE LISTING

<110> BORDIGNON, Matteo
<120> MIA - Melanoma Inhibitory Activity inhibitors for detecting,
   preventing and curing vitiligo
<130> RBW12217-PCT
<150> ITRM2011A000134
   <151> 2011-03-22
<160> 49
<170> BiSSAP 1.0
<210> 1
   <211> 7
   <212> PRT
   <213> Artificial Sequence
<220>
   <221> SOURCE
   <222> 1..7
   <223> /mol_type="protein" /note="inhibits the activity of MIA" /organism="Artificial Sequence"
<400> 1
   Val Pro His Ile Pro Pro Asn
   1               5
<210> 2
   <211> 7
   <212> PRT
   <213> Artificial Sequence
<220>
   <221> SOURCE
   <222> 1..7
   <223> /mol_type="protein" /note="inhibits the activity of MIA" /organism="Artificial Sequence"
<400> 2
   Met Pro Pro Thr Gln Val Ser
   1               5
<210> 3
   <211> 7
   <212> PRT
   <213> Artificial Sequence
<220>
   <221> SOURCE
   <222> 1..7
   <223> /mol_type="protein" /note="inhibits the activity of MIA" /organism="Artificial Sequence"
<400> 3
   Gln Met His Pro Trp Pro Pro
   1               5
<210> 4
   <211> 7
   <212> PRT
   <213> Artificial Sequence
<220>
   <221> SOURCE
   <222> 1..7
   <223> /mol_type="protein" /note="inhibits the activity of MIA" /organism="Artificial Sequence"
<400> 4
   Gln Pro Pro Phe Trp Gln Phe
   1               5
<210> 5
   <211> 7
   <212> PRT
   <213> Artificial Sequence
<220>
   <221> SOURCE
   <222> 1..7
   <223> /mol_type="protein" /note="inhibits the activity of MIA" /organism="Artificial Sequence"
<400> 5
   Thr Pro Pro Gln Gly Leu Ala
   1               5
<210> 6
   <211> 7
   <212> PRT
   <213> Artificial Sequence
<220>
   <221> SOURCE
   <222> 1..7
   <223> /mol_type="protein" /note="inhibits the activity of MIA" /organism="Artificial Sequence"
<400> 6
   Ile Pro Pro Tyr Asn Thr Leu
   1 5
<210> 7
   <211> 7
   <212> PRT
   <213> Artificial Sequence
<220>
   <221> SOURCE
   <222> 1..7
   <223> /mol_type="protein" /note="inhibits the activity of MIA" /organism="Artificial Sequence"
<400> 7
   Ala Val Arg Pro Ala Pro Leu
   1 5
<210> 8
   <211> 7
   <212> PRT
   <213> Artificial Sequence
<220>
   <221> SOURCE
   <222> 1..7
   <223> /mol_type="protein" /note="inhibits the activity of MIA" /organism="Artificial Sequence"
<400> 8
   Gly Ala Lys Pro His Pro Gln
   1 5
<210> 9
   <211> 7
   <212> PRT
   <213> Artificial Sequence
<220>
   <221> SOURCE
   <222> 1..7
   <223> /mol_type="protein" /note="inhibits the activity of MIA" /organism="Artificial Sequence"
<400> 9
   Gln Gln Leu Ser Pro Leu Pro
   1 5
<210> 10
   <211> 7
   <212> PRT
   <213> Artificial Sequence
<220>
   <221> SOURCE
   <222> 1..7
   <223> /mol_type="protein" /note="inhibits the activity of MIA" /organism="Artificial Sequence"
<400> 10
   Gly Pro Pro Pro Ser Pro Val
   1               5
<210> 11
   <211> 7
   <212> PRT
   <213> Artificial Sequence
<220>
   <221> SOURCE
   <222> 1..7
   <223> /mol_type="protein" /note="inhibits the activity of MIA" /organism="Artificial Sequence"
<400> 11
   Leu Pro Leu Thr Pro Leu Pro
   1               5
<210> 12
   <211> 12
   <212> PRT
   <213> Artificial Sequence
<220>
   <221> SOURCE
   <222> 1..12
   <223> /mol_type="protein" /note="inhibits the activity of MIA" /organism="Artificial Sequence"
<400> 12
   Gln Leu Asn Val Asn His Gln Ala Arg Ala Asp Gln
   1               5                   10
<210> 13
   <211> 12
   <212> PRT
   <213> Artificial Sequence
<220>
   <221> SOURCE
   <222> 1..12
   <223> /mol_type="protein" /note="inhibits the activity of MIA" /organism="Artificial Sequence"
<400> 13
   Thr Ser Ala Ser Thr Arg Pro Glu Leu His Tyr Pro
   1               5                   10
<210> 14
   <211> 12
   <212> PRT
   <213> Artificial Sequence
<220>
   <221> SOURCE
   <222> 1..12
   <223> /mol_type="protein" /note="inhibits the activity of MIA" /organism="Artificial Sequence"
<400> 14
   Thr Phe Leu Pro His Gln Met His Pro Trp Pro Pro
   1               5                   10
<210> 15
   <211> 12
   <212> PRT
   <213> Artificial Sequence
<220>
   <221> SOURCE
   <222> 1..12
   <223> /mol_type="protein" /note="inhibits the activity of MIA" /organism="Artificial Sequence"
<400> 15
   Val Pro His Ile Pro Pro Asn Ser Met Ala Leu Thr
   1               5                   10
<210> 16
   <211> 12
   <212> PRT
   <213> Artificial Sequence
<220>
   <221> SOURCE
   <222> 1..12
   <223> /mol_type="protein" /note="inhibits the activity of MIA" /organism="Artificial Sequence"
<400> 16
   Arg Leu Thr Leu Leu Val Leu Ile Met Pro Ala Pro
   1               5                   10
<210> 17
   <211> 12
   <212> PRT
   <213> Artificial Sequence
<220>
   <221> SOURCE
   <222> 1..12
   <223> /mol_type="protein" /note="inhibits the activity of MIA" /organism="Artificial Sequence"
<400> 17
   Tyr Asn Leu Pro Lys Val Ser Ser Asn Leu Ser Pro
   1               5                   10
<210> 18
   <211> 12
   <212> PRT
   <213> Artificial Sequence
<220>
   <221> SOURCE
   <222> 1..12
   <223> /mol_type="protein" /note="inhibits the activity of MIA" /organism="Artificial Sequence"
<400> 18
   Met Pro Pro Thr Gln Val Ser Lys Phe Arg Leu Ile
   1               5                   10
<210> 19
   <211> 12
   <212> PRT
   <213> Artificial Sequence
<220>
   <221> SOURCE
   <222> 1..12
   <223> /mol_type="protein" /note="inhibits the activity of MIA" /organism="Artificial Sequence"
<400> 19
   Ala Asn Ile Asp Ala Thr Pro Leu Phe Leu Arg Ala
   1               5                   10
<210> 20
   <211> 12
   <212> PRT
   <213> Artificial Sequence
<220>
   <221> SOURCE
   <222> 1..12
   <223> /mol_type="protein" /note="inhibits the activity of MIA" /organism="Artificial Sequence"
<400> 20
   Leu Leu Arg Thr Thr Glu Thr Leu Pro Met Phe Leu
   1               5                   10
<210> 21
   <211> 7
   <212> PRT
   <213> Artificial Sequence
<220>
   <221> SOURCE
   <222> 1..7
   <223> /mol_type="protein" /note="inhibits the activity of MIA" /organism="Artificial Sequence"
<400> 21
   Ser Ala Leu Glu Pro Leu Val
   1               5
<210> 22
   <211> 7
   <212> PRT
   <213> Artificial Sequence
<220>
   <221> SOURCE
   <222> 1..7
   <223> /mol_type="protein" /note="inhibits the activity of MIA" /organism="Artificial Sequence"
<400> 22
   Gly Ser Pro Thr Pro Asn Ala
   1               5
<210> 23
   <211> 7
   <212> PRT
   <213> Artificial Sequence
<220>
   <221> SOURCE
   <222> 1..7
   <223> /mol_type="protein" /note="inhibits the activity of MIA" /organism="Artificial Sequence"
<400> 23
   Ala Pro Ser His Ala Thr His
   1               5
<210> 24
   <211> 7
   <212> PRT
   <213> Artificial Sequence
<220>
   <221> SOURCE
   <222> 1..7
   <223> /mol_type="protein" /note="inhibits the activity of MIA" /organism="Artificial Sequence"
<400> 24
   Thr Thr Val Gly His Ser Asp
   1               5
<210> 25
   <211> 7
   <212> PRT
   <213> Artificial Sequence
<220>
   <221> SOURCE
   <222> 1..7
   <223> /mol_type="protein" /note="inhibits the activity of MIA" /organism="Artificial Sequence"
<400> 25
   Thr His Phe Ser Thr Phe Thr
   1               5
<210> 26
   <211> 7
   <212> PRT
   <213> Artificial Sequence
<220>
   <221> SOURCE
   <222> 1..7
   <223> /mol_type="protein" /note="inhibits the activity of MIA" /organism="Artificial Sequence"
<400> 26
   Ser Leu Leu Leu Asp Thr Ser
   1               5
<210> 27
   <211> 12
   <212> PRT
   <213> Artificial Sequence
<220>
   <221> SOURCE
   <222> 1..12
   <223> /mol_type="protein" /note="inhibits the activity of MIA" /organism="Artificial Sequence"
<400> 27
   Ser Val Ala Met Lys Ala His Lys Pro Leu Leu Pro
   1               5                   10
<210> 28
   <211> 12
   <212> PRT
   <213> Artificial Sequence
<220>
   <221> SOURCE
   <222> 1..12
   <223> /mol_type="protein" /note="inhibits the activity of MIA" /organism="Artificial Sequence"
<400> 28
   Asn Thr Ile Pro Gly Phe Ala Ser Lys Ser Leu Asp
   1               5                   10
<210> 29
   <211> 12
   <212> PRT
   <213> Artificial Sequence
<220>
   <221> SOURCE
   <222> 1..12
   <223> /mol_type="protein" /note="inhibits the activity of MIA" /organism="Artificial Sequence"
<400> 29
   Val Ser Asn Tyr Lys Phe Tyr Ser Thr Thr Ser Ser
   1 5 10
<210> 30
   <211> 12
   <212> PRT
   <213> Artificial Sequence
<220>
   <221> SOURCE
   <222> 1..12
   <223> /mol_type="protein" /note="inhibits the activity of MIA" /organism="Artificial Sequence"
<400> 30
   Val Ser Arg His Gln Ser Trp His Pro His Asp Leu
   1 5 10
<210> 31
   <211> 12
   <212> PRT
   <213> Artificial Sequence
<220>
   <221> SOURCE
   <222> 1..12
   <223> /mol_type="protein" /note="inhibits the activity of MIA" /organism="Artificial Sequence"
<400> 31
   His Leu Asn Ile Leu Ser Thr Leu Trp Lys Tyr Arg
   1 5 10
<210> 32
   <211> 12
   <212> PRT
   <213> Artificial Sequence
<220>
   <221> SOURCE
   <222> 1..12
   <223> /mol_type="protein" /note="inhibits the activity of MIA" /organism="Artificial Sequence"
<400> 32
   His Asn Ala Ser Pro Ser Trp Gly Ser Pro Val Met
   1 5 10
<210> 33
   <211> 12
   <212> PRT
   <213> Artificial Sequence
<220>
   <221> SOURCE
   <222> 1..12
   <223> /mol_type="protein" /note="inhibits the activity of MIA" /organism="Artificial Sequence"
<400> 33
   Ser His Pro Trp Asn Ala Gln Arg Glu Leu Ser Val
   1 5 10
<210> 34
   <211> 12
   <212> PRT
   <213> Artificial Sequence
<220>
   <221> SOURCE
   <222> 1..12
   <223> /mol_type="protein" /note="inhibits the activity of MIA" /organism="Artificial Sequence"
<400> 34
   His His Trp Pro Phe Trp Arg Thr Leu Pro Leu Ser
   1 5 10
<210> 35
   <211> 12
   <212> PRT
   <213> Artificial Sequence
<220>
   <221> SOURCE
   <222> 1..12
   <223> /mol_type="protein" /note="inhibits the activity of MIA" /organism="Artificial Sequence"
<400> 35
   Trp His Thr Lys Phe Leu Pro Arg Tyr Leu Pro Ser
   1 5 10
<210> 36
   <211> 12
   <212> PRT
   <213> Artificial Sequence
<220>
   <221> SOURCE
   <222> 1..12
   <223> /mol_type="protein" /note="inhibits the activity of MIA" /organism="Artificial Sequence"
<400> 36
   Asn Asn Thr Ser Phe Thr Val Val Pro Ser Val Pro
   1 5 10
<210> 37
   <211> 12
   <212> PRT
   <213> Artificial Sequence
<220>
   <221> SOURCE
   <222> 1..12
   <223> /mol_type="protein" /note="inhibits the activity of MIA" /organism="Artificial Sequence"
<400> 37
   Ser His Leu Ser Thr Trp Lys Trp Trp Gln Asn Arg
   1 5 10
<210> 38
   <211> 12
   <212> PRT
   <213> Artificial Sequence
<220>
   <221> SOURCE
   <222> 1..12
   <223> /mol_type="protein" /note="inhibits the activity of MIA" /organism="Artificial Sequence"
<400> 38
   Phe His Trp His Pro Arg Leu Trp Pro Leu Pro Ser
   1 5 10
<210> 39
   <211> 12
   <212> PRT
   <213> Artificial Sequence
<220>
   <221> SOURCE
   <222> 1..12
   <223> /mol_type="protein" /note="inhibits the activity of MIA" /organism="Artificial Sequence"
<400> 39
   Trp His Trp Thr Tyr Gly Trp Arg Pro Pro Ala Met
   1 5 10
<210> 40
   <211> 12
   <212> PRT
   <213> Artificial Sequence
<220>
   <221> SOURCE
   <222> 1..12
   <223> /mol_type="protein" /note="inhibits the activity of MIA" /organism="Artificial Sequence"
<400> 40
   Phe His Trp Arg Tyr Pro Leu Pro Leu Pro Gly Gln
   1 5 10
<210> 41
   <211> 12
   <212> PRT
   <213> Artificial Sequence
<220>
   <221> SOURCE
   <222> 1..12
   <223> /mol_type="protein" /note="inhibits the activity of MIA" /organism="Artificial Sequence"
<400> 41
   Trp His Trp Pro Leu Phe Ile Pro Asn Thr Thr Ala
   1               5                   10
<210> 42
   <211> 12
   <212> PRT
   <213> Artificial Sequence
<220>
   <221> SOURCE
   <222> 1..12
   <223> /mol_type="protein" /note="inhibits the activity of MIA" /organism="Artificial Sequence"
<400> 42
   Trp His Asn Gly Ile Trp Trp His Tyr Gly Val Arg
   1               5                   10
<210> 43
   <211> 12
   <212> PRT
   <213> Artificial Sequence
<220>
   <221> SOURCE
   <222> 1..12
   <223> /mol_type="protein" /note="inhibits the activity of MIA" /organism="Artificial Sequence"
<400> 43
   His His Leu Asn Tyr Leu Trp Pro Trp Thr Arg Val
   1               5                   10
<210> 44
   <211> 12
   <212> PRT
   <213> Artificial Sequence
<220>
   <221> SOURCE
   <222> 1..12
   <223> /mol_type="protein" /note="inhibits the activity of MIA" /organism="Artificial Sequence"
<400> 44
   Phe Trp His Arg Trp Ser Thr Phe Pro Glu Gln Pro
   1               5                   10
<210> 45
   <211> 12
   <212> PRT
   <213> Artificial Sequence
<220>
   <221> SOURCE
   <222> 1..12
   <223> /mol_type="protein" /note="inhibits the activity of MIA" /organism="Artificial Sequence"
<400> 45
   Trp His Met Ser Tyr Phe Trp Thr Arg Pro Pro Gln
   1 5 10
<210> 46
   <211> 12
   <212> PRT
   <213> Artificial Sequence
<220>
   <221> SOURCE
   <222> 1..12
   <223> /mol_type="protein" /note="inhibits the activity of MIA" /organism="Artificial Sequence"
<400> 46
   Phe His Leu Asn Trp Pro Ser Arg Ala Asp Tyr Leu
   1 5 10
<210> 47
   <211> 12
   <212> PRT
   <213> Artificial Sequence
<220>
   <221> SOURCE
   <222> 1..12
   <223> /mol_type="protein" /note="inhibits the activity of MIA" /organism="Artificial Sequence"
<400> 47
   Trp His Lys Asn Thr Asn Trp Pro Trp Arg Thr Leu
   1 5 10
<210> 48
   <211> 12
   <212> PRT
   <213> Artificial Sequence
<220>
   <221> SOURCE
   <222> 1..12
   <223> /mol_type="protein" /note="inhibits the activity of MIA" /organism="Artificial Sequence"
<400> 48
   Ala Leu Ser Pro Ser Gln Ser His Pro Val Arg Ser
   1 5 10
<210> 49
   <211> 12
   <212> PRT
   <213> Artificial Sequence
<220>
   <221> SOURCE
   <222> 1..12
   <223> /mol_type="protein" /note="inhibits the activity of MIA" /organism="Artificial Sequence"
<400> 49
   Gly Thr Gln Ser Thr Ala Ile Pro Ala Pro Thr Asp
   1               5                   10